# FASCICULE DE BREVET EUROPEEN

(11) **EP 3 307 209 B1**
(45) Date de publication et mention de la délivrance du brevet: **27.05.2020**
(21) Numéro de dépôt: 16738481.7
(22) Date de dépôt: 13.06.2016
(51) Int. Cl.: A61F 2/64

(54) **DISPOSITIF DE VERROUILLAGE DEVERROUILLAGE D'UNE PROTHESE DE GENOU**
VORRICHTUNG ZUM SPERREN UND ENTSPERREN EINER KNIEPROTHESE
MEANS FOR LOCKING AND UNLOCKING A KNEE PROSTHESIS

(30) Priorité: 12.06.2015 FR 1555376
(43) Date de publication de la demande: 18.04.2018
(73) Titulaire: Blanc, Henri, 33850 Leognan (FR)
(72) Inventeur: Blanc, Henri, 33850 Leognan (FR)
(74) Mandataire: Aquinov
(86) Numéro de dépôt international: PCT/FR2016/051420
(87) Numéro de publication internationale: WO 2016/198808

(56) Documents cités:
- EP-A2- 2 502 607
- GB-A- 344 430
- US-B1- 7 087 091
- US-B1- 8 715 367

## Description

La présente invention concerne un dispositif de verrouillage / déverrouillage d'une prothèse de genou, fonctionnant avec la seule énergie du porteur, notamment utilisable en cas d'amputation fémorale.

Il existe de nombreux dispositifs sophistiqués utilisant des systèmes de vérins hydrauliques avec des électrovannes pilotées à partir d'informations données par des capteurs, intégrant des microprocesseurs. Du fait de la présence de systèmes utilisant des moyens électriques, il est nécessaire de prévoir une source d'énergie électrique et un rechargement de ladite source. Ces dispositifs sophistiqués permettent d'obtenir des réglages très précis mais ils présentent de nombreux inconvénients, notamment ils sont complexes et d'un coût élevé voire très élevé. Dans les pays possédant une infrastructure médicale développée, les amputations existent notamment suite à des accidents ou des atteintes vasculaires chroniques associées à un diabète et on constate que certaines personnes ayant un faible potentiel musculaire sont en demande d'une marche plus sécurisée.

Dans les pays présentant une infrastructure médicale limitée, l'accès à des prothèses sophistiquées est encore plus difficile.

On connaît par le brevet français FR 2 972 920 un dispositif de contrôle d'une prothèse de genou pour jambe.

Ce dispositif de contrôle est entièrement mécanique et d'une grande compacité, il est d'un entretien très limité et d'un coût réduit permettant un accès plus aisé sur le plan financier. Un tel dispositif de contrôle est adapté pour des applications en grand nombre afin d'appareiller notamment des enfants.

Pour atteindre cet objectif il est prévu deux parties articulées à rotation entre elles, l'une la partie fémorale solidaire de la partie amputée du fémur, ceci à travers tout moyen adapté et l'autre la partie tibiale qui reçoit un tibia et un pied artificiels, ceci de façon connue.

Le but de cette prothèse de l'art antérieur est d'assurer au moins une liberté de l'articulation à rotation lorsque le porteur soulève la jambe et que celle-ci ne se trouve pas en appui sur le sol et d'assurer un blocage de la rotation lorsque la jambe est en appui sur le sol, pour permettre un basculement du corps vers l'avant, ceci de façon à générer une marche aussi naturelle que possible pour le porteur.

Cette prothèse de l'art antérieur propose une solution intégrant une roue libre et des moyens de contrôle de ladite roue libre. Ces moyens de contrôle sont commandés par un câble monté sur deux poulies avec un ressort de précontrainte et une plaquette mobile qui tend ou détend ledit câble et permet de bloquer ou de libérer ladite roue libre.

Le montage de ces moyens de contrôle à câbles reste délicat et il faut un câble ajusté, un ressort de précontrainte du câble ou des moyens analogues.

Le but de la présente invention est de proposer une solution de contrôle de la roue libre donc du verrouillage / déverrouillage, qui est simple, aisée à fabriquer, d'une grande facilité de montage et qui rend la prothèse qui en est équipée encore plus fiable.

Une variante permet également une mise en station assise ainsi qu'un relevage avec un appui sur la jambe prothétique et une mise en service de la prothèse en mode marche de façon automatique dès la station verticale atteinte.

Le dispositif de verrouillage / déverrouillage d'une prothèse de genou selon la présente invention est maintenant décrit en détail suivant un mode de réalisation préférentiel, non limitatif, ceci en regard des dessins annexés, dessins sur lesquels les différentes figures représentent :
- Figure 1 : une vue schématique en perspective éclatée de la prothèse de genou selon la présente invention, avec son mécanisme de verrouillage / déverrouillage
- Figure 2 : une vue de la prothèse de la figure 1, montée,
- Figure 3 : une vue de la prothèse de la figure 1, avec la joue droite retirée,
- Figures 4A et 4B : deux vues en coupe de la prothèse de la figure 1 montrant la biellette de manœuvre, et l'agencement des pions des moyens de déverrouillage,
- Figures 5A à 5G : des vues schématiques lors des différentes phases de fonctionnement durant la marche du porteur.
- Figure 6 : une vue en perspective éclatée d'une variante de réalisation du mécanisme de verrouillage / déverrouillage de la prothèse,
- Figure 7 : une vue de devant, en perspective, après assemblage,
- Figure 8 : une vue de derrière, en perspective, après assemblage,
- Figure 9 : une vue en coupe, de la partie rotulienne de la prothèse,
- Figures 10A et 10B : une vue schématique de l'agencement et du fonctionnement de relevage avec excentrique, une fois assis, applicable aux deux variantes.

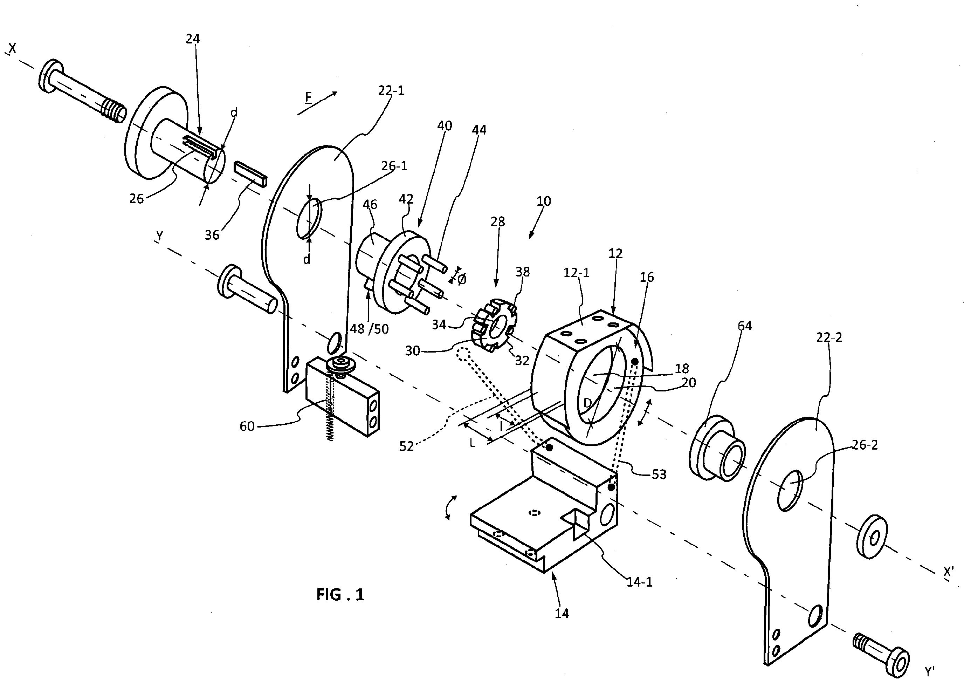

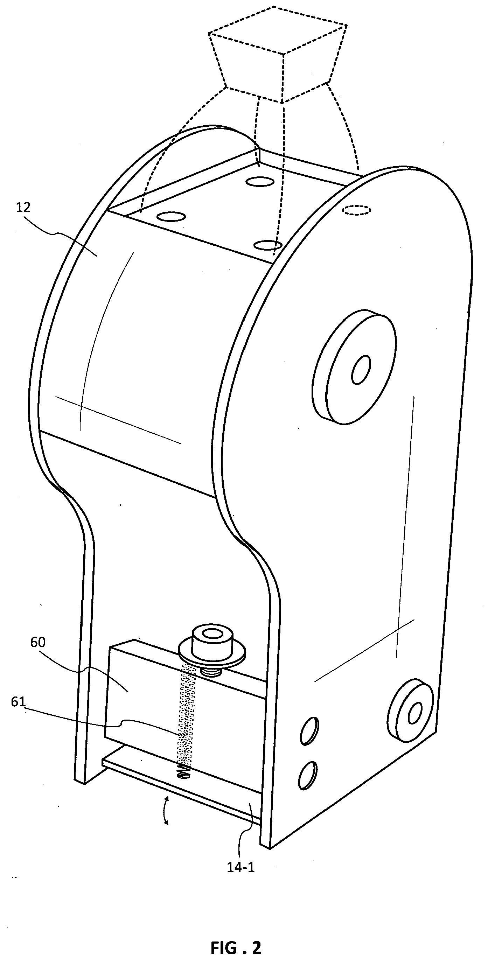

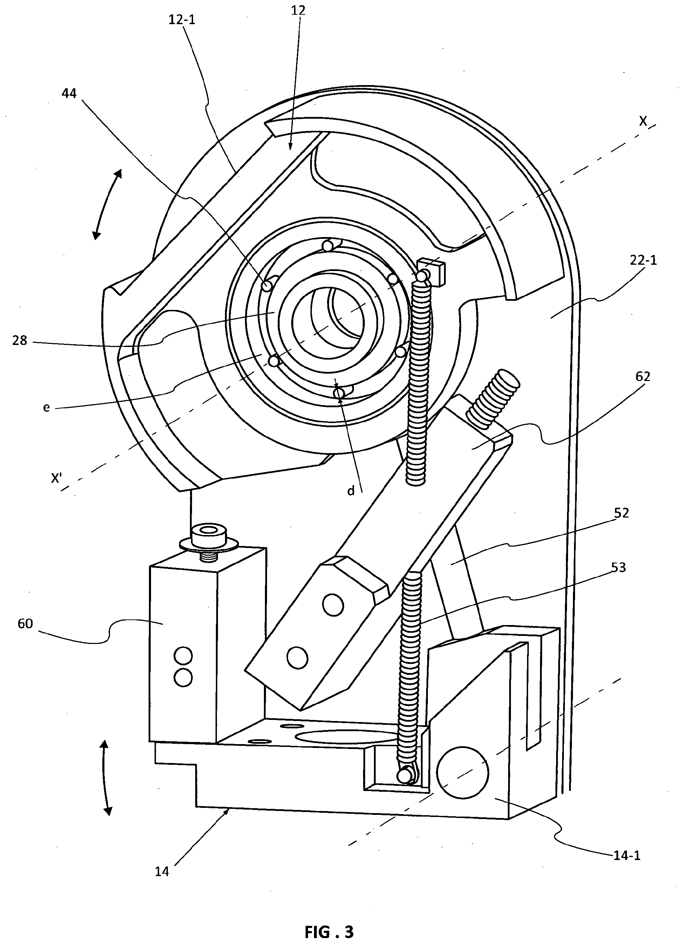

Sur les figures 1, 2 et 3, on a représenté le dispositif de verrouillage / déverrouillage d'une prothèse 10 de genou selon la présente invention, cette prothèse 10 comprenant une partie 12 fémorale et une partie 14 tibiale.

La partie 12 fémorale est prévue pour être reliée à la cuisse du porteur, non représentée, par tout moyen adapté, non inclus dans la présente invention, seule une platine 12-1 de fixation fémorale et une platine 14-1 de fixation tibiale étant prévues pour les liaisons mécaniques nécessaires avec ces moyens adaptés.

Les parties 12 fémorale et 14 tibiale sont montées toutes les deux à rotation, coaxiales autour d'un axe XX', correspondant à l'axe de pliage naturel du genou.

La partie 12 fémorale comprend une pièce cylindrique 16, d'une largeur L, comportant tous les évidements et rognages nécessaires pour l'alléger, dont l'axe central est coaxial avec l'axe XX'.

La partie supérieure de ce disque porte la platine 12-1 de fixation destinée à recevoir des moyens de liaison à la cuisse du porteur comme indiqué ci-avant, en l'occurrence, ces moyens de fixation consistant en un méplat ménagé sur la surface radiale extérieure supérieure de la pièce cylindrique 16, comportant des alésages taraudés destinés à recevoir des vis par exemple.

Cette pièce cylindrique 16 comprend un logement 18, central, cylindrique, ménagé de façon coaxiale à l'axe XX'.

Ce logement 18 central, cylindrique, comporte donc une piste 20 d'une largeur l, inférieure à la largeur L la pièce cylindrique 16 et d'un diamètre D.

La partie 14 tibiale comprend notamment deux joues 22-1 et 22-2, gauche et droite, ainsi qu'un arbre 24 traversant, unique, monté dans des trous 26-1 et 26-2 correspondants, ménagés dans lesdites joues, cet arbre 24 traversant unique étant également coaxial à l'axe XX'.

Cet arbre 24 traversant présente un diamètre d, inférieur à D, libérant un espace annulaire E. Cet arbre 24 traversant comporte une cannelure 26, longitudinale, disposée suivant une génératrice.

Entre la périphérie de cet arbre 24 traversant et la piste 20 du disque , on trouve un montage 28 à roue libre en l'occurrence un montage de roue libre à rouleaux.

Ce montage 28 à roue libre comprend un pignon 30 muni de rampes 32, portant également une cannelure 34 de profil conjugué de la cannelure 26 de l'arbre 24 traversant, une clavette 36 assurant l'immobilisation angulaire dudit pignon 30 à rampes 32 sur ledit arbre 24 traversant.

Le pignon 30 à rampes et l'arbre 24 traversant sont donc également coaxiaux avec l'axe XX'. Après montage, la périphérie des rampes 32 libère un espace e inférieur à E.

Un rouleau 38 est interposé entre chaque rampe dudit pignon et la piste 20 du disque, le diamètre de ces rouleaux étant supérieur à la distance séparant la partie haute de la rampe et ladite piste 20 et inférieur à la distance séparant la partie basse de la rampe de cette même piste 20.

Dans un sens chaque rouleau peut se coincer entre sa rampe 32 et la piste 20, assurant une liaison mécanique temporaire en rotation entre le disque et la partie tibiale. Dans l'autre sens, chaque rouleau coincé se décoince et libère la rotation entre le disque et la partie tibiale.

Il est aussi prévu des moyens 40 de déverrouillage permanent des rouleaux 38, comme cela est visible sur les figures 1, 2 et 3.

Ces moyens 40 de déverrouillage comprennent une roue 42 à pions 44, montée également coaxiale à l'axe XX'. Le nombre de pions 44 est égal au nombre de rampes 32 et de rouleaux 38.

Cette roue 42 à pions 44 est montée libre à rotation autour de l'arbre 24 traversant.

Les pions 44 sont orientés parallèlement à des génératrices dudit arbre 24 traversant.

Ces pions 44, de façon préférentielle sous forme de cylindre, ne sont supportés que par une extrémité et ils pénètrent dans l'espace annulaire e, situé entre la périphérie extérieure des rampes 32 et la piste 20. Le diamètre ∅ de ces pions est inférieur à la dimension e.

Ces pions ont une longueur telle qu'ils viennent au droit des rampes sans excéder leur largeur. Cette roue 42 à pions 44 comprend une partie 46 déportée entre la pièce cylindrique 16 et la joue gauche 22-1.

Cette partie 46 déportée, monolithique, reçoit quant à elle, des moyens 48 de commande angulaire.

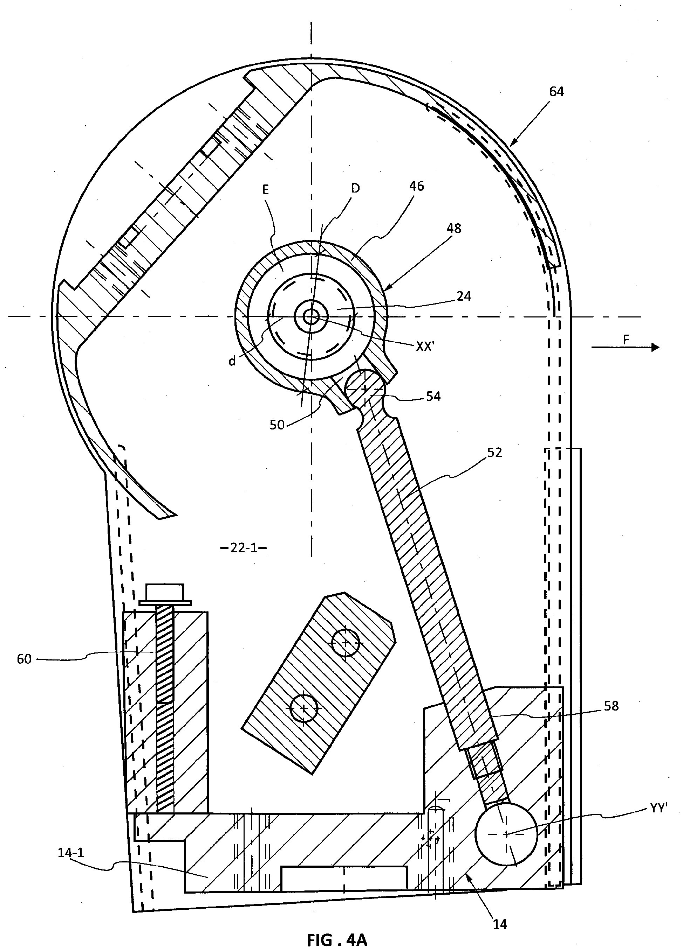

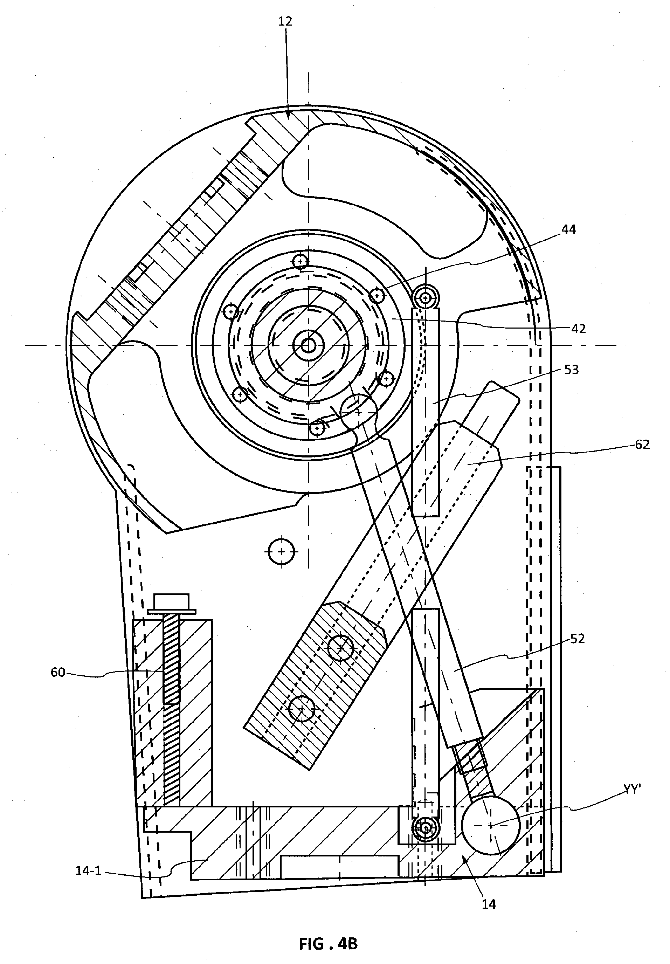

Ces moyens 48 de commande angulaire, comme représenté notamment sur la figure 4A, comprennent un logement 50 rotulien borgne, une biellette 52 avec une tête 54 apte à coopérer sous forme d'une articulation à rotule avec le logement 50 rotulien borgne.

Le logement 50 rotulien borgne présente une orientation radiale.

La biellette 52 se prolonge vers la platine 14-1 de fixation tibiale.

Des moyens 53 de rappel, en l'occurrence un ressort, sont interposés entre la partie 12 fémorale et la partie 14 tibiale. En l'absence d'efforts autres, notamment d'inertie, ce ressort 53 rappelle en permanence les deux parties fémorale et tibiale en alignement. Le ressort pourrait être un vérin oléopneumatique avec un réglage de l'effort de rappel.

La partie 14 tibiale comporte donc les joues 22-1 et 22-2 et pivote autour de l'axe XX' et de la pièce cylindrique 16 solidaire de la partie fémorale par l'intermédiaire de l'arbre 24 traversant. La platine 14-1 tibiale est équipée de moyens de liaison destinés à recevoir un tibia artificiel, ces moyens de fixation consistant en un méplat ménagé sur la surface inférieure de ladite platine, comportant des alésages taraudés destinés à recevoir des vis par exemple.

Cette platine 14-1 de fixation tibiale est articulée par un bord distal autour d'un axe YY' défini par un arbre 56, axe de rotation parallèle à l'axe XX' et disposé en avant de l'axe XX' par rapport au sens de la marche indiqué par la flèche F. Ceci sera explicité dans la description du fonctionnement.

Cette platine 14-1 comprend un trou borgne 58, orienté radialement par rapport audit axe YY', destiné à recevoir de façon rigide la seconde extrémité de la biellette 52. Ce trou borgne 58 reçoit la biellette par vissage par exemple, ledit trou borgne 58 étant alors taraudé et l'extrémité de la biellette étant filetée, de façon à permettre un ajustement en longueur également.

Une butée 60, réglable, solidaire des joues 22-1 et 22-2, est disposée sur le bord proximal de façon à limiter l'angle de pivotement de la platine 14-1 vers le haut et donc le mouvement de la roue 42 à pions à laquelle elle est liée par la biellette 52. Cette butée porte une vis qui permet d'affiner le réglage de butée ainsi que des systèmes d'amortissement du choc lors du contact avec la partie tibiale 14.

Cette butée 60 est équipée d'un ressort 61 de rappel qui repousse la platine 14-1 vers le bas et qui assure la libération de la roue libre à rouleaux comme cela sera précisé plus avant lors de la description du fonctionnement.

De même, l'amplitude angulaire de la platine 14-1 est limitée vers le bas afin de conférer à ladite platine une liberté angulaire de quelques degrés.

Le dispositif selon la présente invention comprend de plus des moyens 62 amortisseurs et butée, sous forme d'un bloc muni de pistons à ressorts par exemple. Ces moyens 62 amortisseurs sont utiles de façon à diminuer le choc au contact et à éviter le mouvement angulaire du genou au-delà de l'alignement de la partie fémorale et de la partie tibiale. Ces moyens ne sont pas représentés sur la figure 1 pour la clarté du graphisme.

En effet, il faut que la partie 14 tibiale fasse toujours un angle inférieur ou égal à 180°, comme au naturel.

De même, une bague 64 entretoise assure le complément avec la joue.

On note aussi que cette bague 64 assure avec la roue 42, un centrage des rouleaux, latéralement. Il serait aussi possible de remplacer les rouleaux par des billes. Dans ce cas, la question du centrage ne se poserait pas. La portée d'une bille reste un point, ce qui peut être largement suffisant pour l'application selon la présente invention, mais dans le cadre d'une recherche de fiabilité, un rouleau présente une ligne de contact, à savoir une génératrice périphérique dudit rouleau, si bien que l'effort est plus réparti, ce qui en fait le choix préférentiel dans la présente invention.

En variante, on peut aussi disposer des roulements à billes dans l'espace libre afin d'améliorer le centrage et le support du pignon 30 par rapport à la piste 20, tout en tenant compte du fait que la prothèse doit présenter un poids limité.

Ceci permet une diminution des frottements, des jeux de fonctionnement et des usures liées aux mouvements du genou qui se comptent en très grand nombre dès lors que le sujet appareillé commence à marcher.

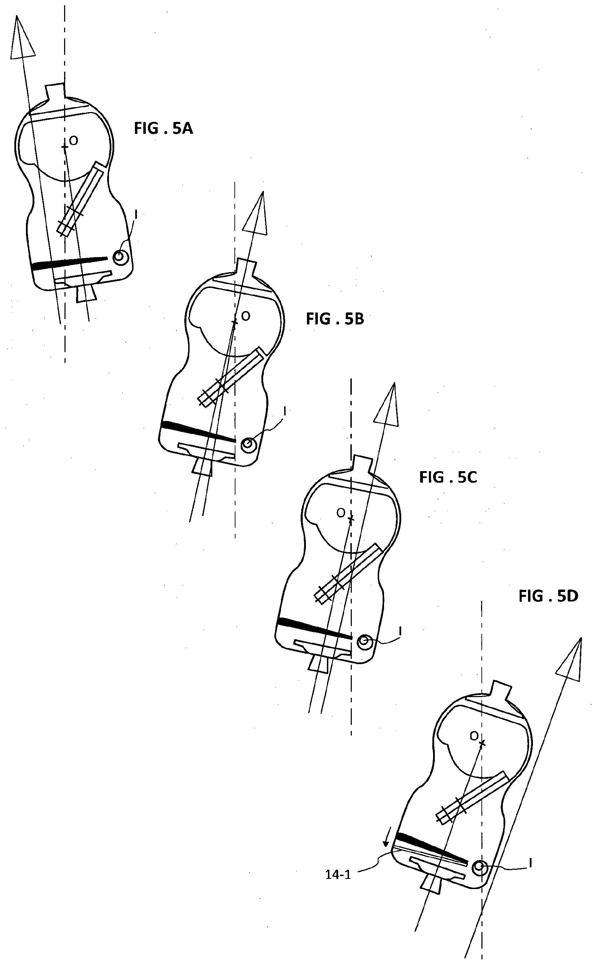

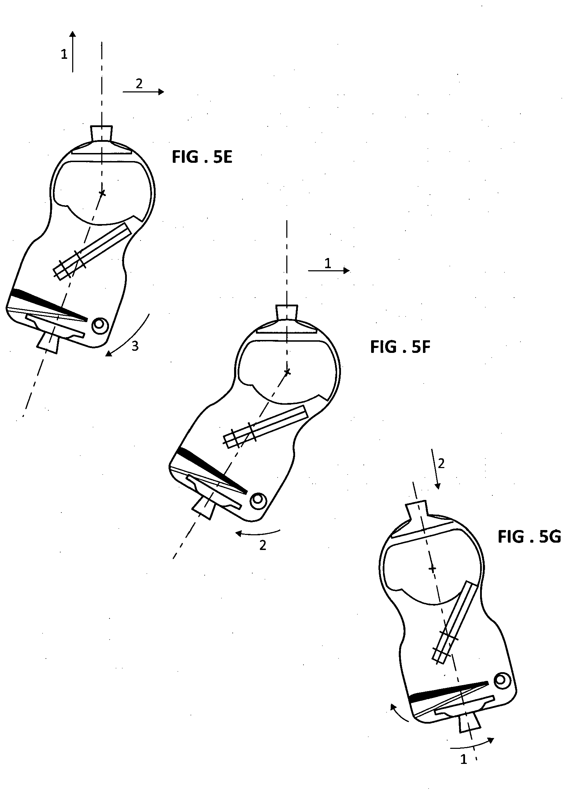

Le fonctionnement du dispositif de verrouillage / déverrouillage suivant l'invention est maintenant décrit en regard du synoptique de la figure 5.

Sur la figure 5A, le porteur est en position avec la jambe en appui au sol. La platine 14-1 est pivotée vers le haut autour de l'axe YY', en appui sur la butée 60. Le ressort 61 de rappel est comprimé, la roue libre est active et verrouille la rotation.

La partie fémorale et la partie tibiale sont immobilisées dans le sens de la fermeture du genou prothétique. La réaction du sol sous le poids du porteur de la prothèse est symbolisée par la flèche qui se situe en arrière du point O et du point I.

La flèche est donc en arrière du point O et de l'axe YY' ou du point I et sensiblement parallèle à l'axe passant par le point O.

Dès lors que, comme sur la figure 5B, le porteur avance, il fait porter le poids sur l'avant et se trouve en appui unipodal, côté prothèse.

Le porteur est toujours en appui au sol et la roue libre est toujours active donc les deux parties restent immobilisées avec un alignement imposé de la partie fémorale et tibiale, figure 5C.

Par contre l'ensemble a pivoté du fait du basculement du corps du porteur vers l'avant si bien que la flèche passe devant le point O.

Le déplacement du porteur de prothèse se poursuit conduisant au diagramme des efforts de la figure 5D.

La réaction du sol s'incline vers l'avant puisque la réaction s'effectue de l'arrière vers l'avant.

La flèche correspondante, symbolisant cette réaction, s'éloigne du point O vers l'avant en dépassant l'axe YY' et donc le point I.

C'est la dernière phase de propulsion vers l'avant juste avant la situation du double appui.

La platine 14-1 est pivotée vers le bas autour de cet axe YY' par le ressort 61 de la butée 60, la biellette 52 actionne la roue 42 avec ses pions 44 qui assurent à leur tour un déverrouillage des rouleaux 38 : la roue libre est désactivée.

Dès lors, le porteur passe dans une situation où la dynamique de la marche va le conduire à ramener sa jambe et c'est donc le début de la phase pendulaire ou oscillante de la jambe prothétique.

Dès lors, figure 5E, après la poussée, le porteur de la prothèse soulève la partie fémorale mais le ressort 53 assure au début un maintien au sol en maintenant l'alignement.

Néanmoins, l'effort de rappel est rapidement vaincu par les efforts exercés pour une marche enchaînée.

Le pied quitte alors l'appui au sol et la roue libre, déjà libérée, peut œuvrer.

De ce fait, sous l'effet de l'inertie, la partie fémorale avance plus vite que la partie tibiale, si bien que, comme en marche naturelle, les deux parties fémorale et tibiale peuvent pivoter et l'angle de 180° initial diminue fortement.

Plus la marche est rapide, plus il y aura d'inertie si bien que le pliage du genou prothétique sera d'autant plus naturel et aérien.

Sur la figure 5F, on constate aisément ce pliage maximum.

Sur la figure 5G, le porteur de la prothèse ayant avancé la partie fémorale, celle-ci atteint son maximum et la partie tibiale libre revient vers l'avant, sous l'effet de la gravité puis de l'inertie, avec l'assistance du ressort 53 des moyens de rappel.

Lorsque le pied se trouve en position requise, le porteur vient en appui et de nouveau il y a blocage angulaire par verrouillage de la roue libre, jambe tendue.

Le cycle de marche est terminé.

On constate aussi que, durant toute cette phase de ramenée de la partie tibiale, si le porteur venait, pour une quelconque raison, à s'appuyer sur sa jambe appareillée, la mise en contact du pied avec le sol bloquerait immédiatement la roue libre dans la position angulaire dans laquelle elle se trouve, interdisant un pliage supplémentaire et une chute irrémédiable.

La prothèse selon la présente invention est parfaitement sécuritaire, en toute occasion.

Le dispositif selon la présente invention permet un verrouillage / déverrouillage aisé au moyen d'une biellette qui assure le mouvement de rotation de la roue 42 à pions 44 et assure une libération des rouleaux.

Les problèmes d'ajustement sont aisément maîtrisés du fait de cette biellette fixe, vissée, de longueur constante, réglable finement par vissage et les porteurs constatent une marche aisée avec des mouvements extrêmement naturels, sans à-coups, pour une vitesse de marche adaptée.

Le déverrouillage est garanti par le mouvement des pions 44 imposé par la détente du ressort 61 de rappel.

De façon à assurer un verrouillage de façon certaine, les rouleaux sont sollicités, de façon connue, par un ressort, non représenté, qui tend à les pousser vers le haut de la rampe en permanence. Le blocage des rouleaux entre la rampe et la piste est ainsi instantané.

Ces ressorts sont de faible puissance et lesdits ressorts ne peuvent en aucun cas s'opposer aux efforts de poussée des pions 44.

Dans le mode de réalisation préférentiel décrit il a été utilisé une clavette 36 pour immobiliser angulairement le pignon sur l'arbre traversant mais il est aussi possible de recourir à des cannelures qui constituent un moyen tout à fait équivalent.

Ce dispositif de verrouillage / déverrouillage selon l'invention est avantageux et d'une très grande fiabilité.

Le réglage dans ce mode de réalisation s'effectue par les butées si bien que l'adaptation au porteur est rapide et précise.

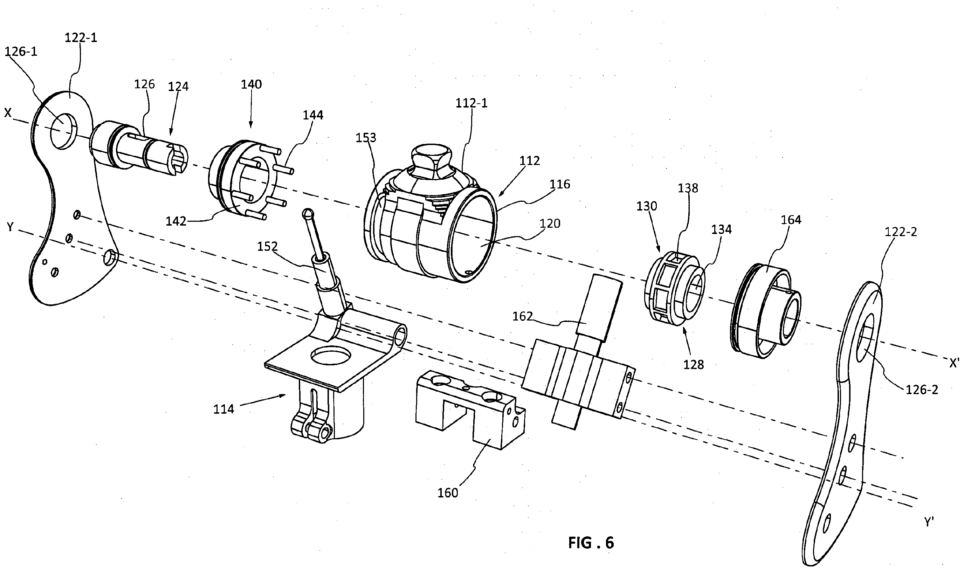

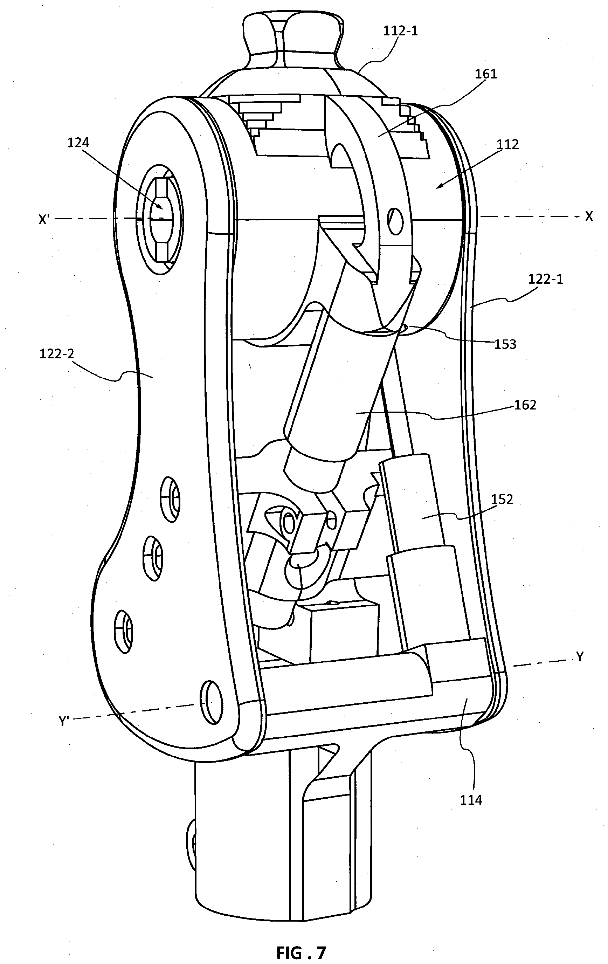

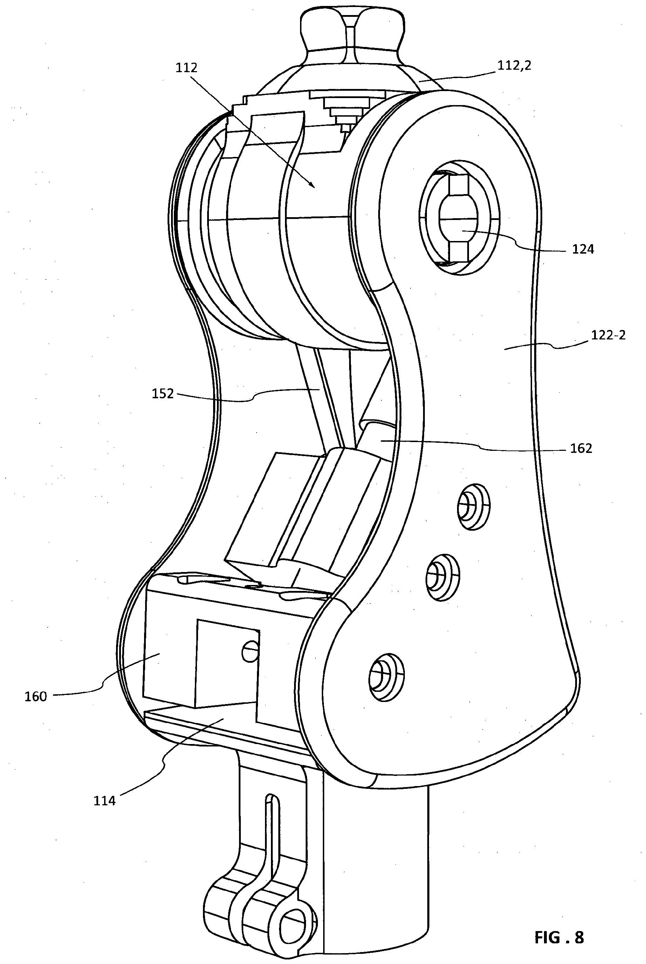

La présente invention prévoit également une variante de réalisation représentée sur les figures 6 à 9.

Cette variante comporte de très nombreuses pièces identiques à celles du mode de réalisation précédemment décrit, ces pièces ne seront pas décrites et porteront les mêmes références augmentées de 100.

Les pièces nouvelles porteront des références propres, au-delà du nombre 100.

Sur les figures 6 à 9, on constate que la partie fémorale 112 comporte quelques modifications de forme et notamment la platine 112-1 et les moyens de liaison à la cuisse du porteur sont directement usinés et venus de fabrication avec ladite partie fémorale.

Les parties 112 fémorale et 114 tibiale sont toujours montées à rotation l'une par rapport à l'autre , coaxiale autour d'un axe XX', correspondant à l'axe du genou.

La partie 114 tibiale reste inchangée à l'exception des formes esthétiques extérieures.

Dans la partie fémorale 112, le changement technique consiste en un arbre traversant 124, qui se prolonge sur la largeur et comporte à son extrémité une rainure 125. Cet axe traverse les deux joues 122-1 et 122-2, gauche et droite. Cet arbre traversant 124 est coaxial avec l'axe XX'.

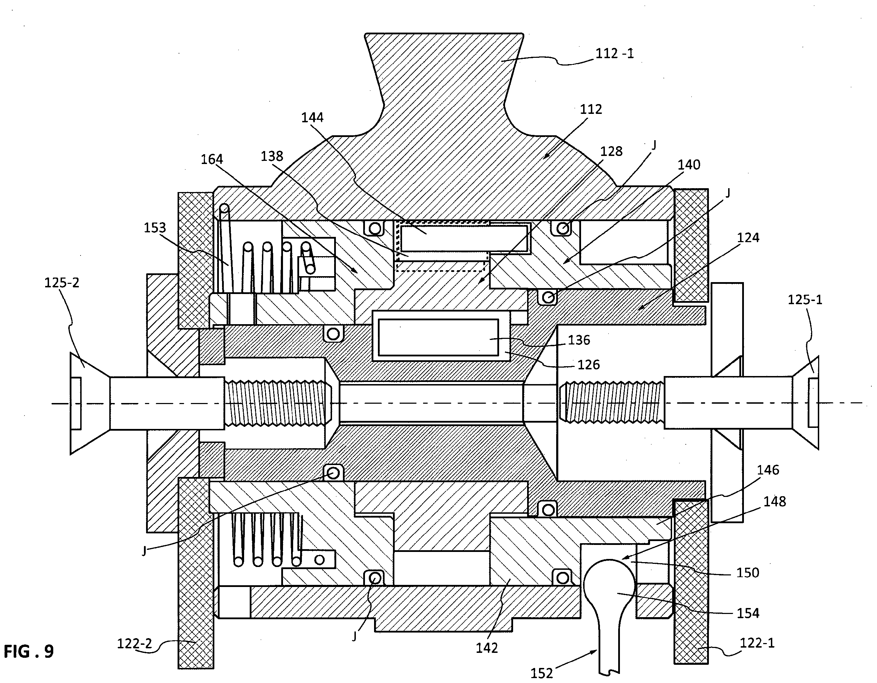

Le montage à roue libre 128 est conservé, en l'occurrence un montage à roue libre à rouleaux 138 reposant sur une rampe 132. Des ressorts peuvent être interposés pour presser les rouleaux sur la rampe, de façon connue et comme dans le mode de réalisation précédent. Ce montage à roue libre 128 est solidaire de l'arbre traversant 124 par une clavette 136 visible sur la figure 9, notamment.

Cette clavette 136 coopère avec une cannelure 134 de profil conjugué d'une cannelure 126 portée par ledit arbre traversant 124.

On retrouve les moyens 140 de déverrouillage permanent des rouleaux 138 sous forme d'une roue 142 à pions 144, montée coaxiale à l'axe XX' et libre en rotation.

Les moyens 148 de commande angulaire sont conservés sensiblement identiques avec un logement 150 rotulien, borgne, une biellette 152 et une tête 154 apte à coopérer avec ledit logement rotulien.

Les moyens 155 de rappel sont modifiés car ils sont sous forme d'un ressort à spires, monté coaxial à l'axe XX'. Les extrémités du ressort sont interposés entre la partie fémorale 112 et l'entretoise 164.

De même, l'arbre 124 traversant reçoit deux vis 125-1 et 125-2 qui assurent le montage des joues et solidarisent lesdites joues 122-1 et 122-2 audit arbre 124. Cet arbre traversant est solidaire de la roue libre 128 comportant les rouleaux 138 et les rampes 132 par la clavette 136.

L'entretoise 164 est également solidaire de l'ensemble et permet le rappel en ouverture de la partie tibiale 114 par rapport à la partie fémorale 112, en l'absence de contraintes ou de blocage.

Les moyens de déverrouillage 140 sont libres en rotation par rapport à l'arbre 124 traversant et les pièces associées, la rotation angulaire étant contrôlée par la biellette 152 dont la tête 154 pénètre dans le logement rotulien 150.

La tige 152 passe à travers une lumière 153, curviligne, ménagée dans la partie fémorale 112 afin de permettre la rotation de ladite partie fémorale sans perturber le fonctionnement de ladite tige 152 de commande des moyens de déverrouillage 140.

Cette lumière curviligne 153 peut comporter une butée réglable angulairement de façon à limiter l'amplitude angulaire du genou, notamment utile en phase de ré-apprentissage de la marche.

La partie fémorale 112 comporte également un bec 161 en avant de la partie purement cylindrique, afin de venir en appui amortissant sur les moyens 162 amortisseurs et butée. Ce bec est intégral avec la partie fémorale 112.

On note la présence de joints toriques J qui sont interposés entre les pièces tournantes de façon à supprimer les pénétrations de poussières et d'eau.

La partie tibiale 114 reste identique à celle du mode de réalisation principal.

L'agencement ainsi décrit se trouve de taille plus réduite et ce plus faible encombrement conduit à la fabrication d'une prothèse de moins grand encombrement et trouve donc une application directe pour les enfants.

Le fonctionnement en mode marche tel que décrit en regard du synoptique de la figure 5 est totalement applicable à cette variante.

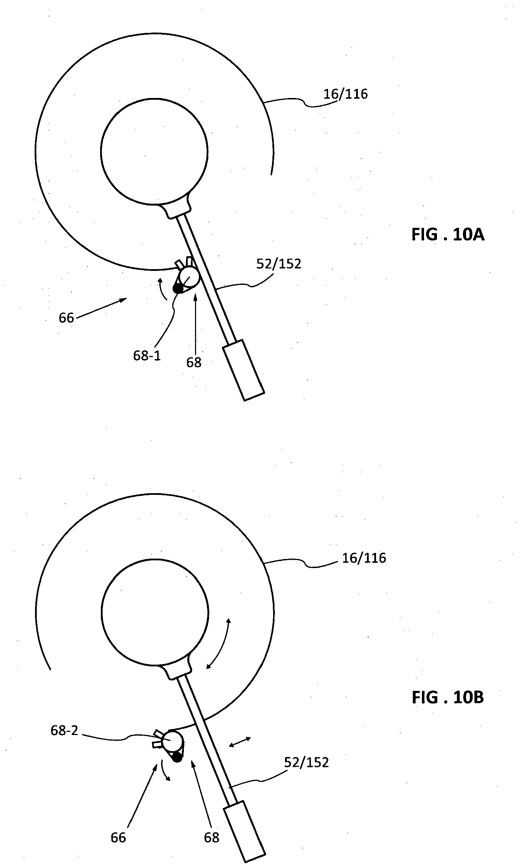

Par contre, il est prévu un perfectionnement applicable aux deux variantes, illustré de façon schématique sur la figure 10.

En effet, lorsque le porteur est assis et que sa jambe équipée de la prothèse est pliée à 90°, le patient qui souhaite se lever ne peut pas prendre appui sur sa jambe prothétique.

Aussi, il est prévu des moyens d'assistance au lever 66 à partir de la position assise de façon à permettre au patient de s'appuyer de façon continue sur sa jambe prothétique dont le genou s'ouvre au fur et à mesure du rapprochement de la station debout.

Ces moyens d'assistance au lever 66 se présentent, par exemple sous forme d'un excentrique 68, apte à prendre deux positions :
- la première position 68-1 d'appui sur la biellette 52, 152 des moyens de déverrouillage pour assurer un déverrouillage permanent par les pions 44, 144 qui repoussent chaque rouleau 38,138 en bas de sa rampe, et
- la seconde position 68-2, sans appui sur la biellette 52, 152 des moyens de déverrouillage 40,140 pour assurer un déverrouillage permanant en laissant la biellette 52,152 des moyens de verrouillage agir en fonctionnement normal, comme sur les figures 5.

Sur les figures 10A et 10B, on a représenté un tel excentrique 68 dans les deux positions. Ainsi, le mouvement de la partie 12, 112 fémorale vient en butée en position assise et actionne l'excentrique par la pièce cylindrique 16, 116 équipée d'un taquet adapté pour mettre ladite biellette dans la première position. Ainsi, au relevage les rouleaux restent déverrouillés. Dans cette position de station debout, la partie fémorale 12, 112 vient sensiblement dans l'alignement de la partie tibiale 14, 114.

La pièce cylindrique 16, 116 par son autre côté, muni d'un autre taquet adapté, assure le retrait de l'excentrique 68. Celui-ci libère alors le fonctionnement de la prothèse sans aucune intervention et le porteur peut alors reprendre sa marche de façon normale avec sa prothèse. On note que dans cette seconde variante, l'arbre traversant permet un montage par empilement autorisant les changements de pièces indépendamment et donc l'entretien des pièces d'usure.

Le montage de l'un des dispositifs décrits de verrouillage / déverrouillage par empilement et emboîtement reste à la portée d'un personnel médical qui ne nécessite ni une grande qualification supplémentaire en mécanique ni un outillage sophistiqué.

Le remplacement de pièces éventuellement usées ou présentant un défaut est très rapide. On note également que les dimensions des pièces sont diminuées si bien que la prothèse elle-même, munie de l'un des dispositifs de verrouillage / déverrouillage est d'un poids réduit. Une telle prothèse, quelle que soit la variante est parfaitement adaptée pour des applications aux enfants.

De plus, le design extérieur des joues et de la pièce fémorale est adaptable très simplement à l'envie esthétique du porteur.

## Revendications

1. Dispositif de verrouillage / déverrouillage d'une prothèse (10,110) de genou, comprenant une partie (12,112) fémorale prévue pour être reliée à la cuisse du porteur, et une partie (14,114) tibiale munie d'une platine (14-1, 114-1) tibiale articulée autour d'un axe YY', apte à recevoir un tibia et un pied artificiels, ces deux parties (12,112) fémorale et (14,114) tibiale étant montées toutes les deux à rotation, coaxiales autour d'un axe XX', les axes XX' et YY' étant parallèles, correspondant à l'axe de pliage du genou naturel, comprenant:
- Une pièce cylindrique (16,116) constituant la partie (12,112) fémorale, avec un logement (18,118), central, cylindrique, ménagé de façon coaxiale à l'axe XX' et comportant une piste (20, 120),
- Deux joues (22-1, 22-2; 122-1, 122-2), gauche et droite et un arbre (24,124) traversant, unique, coaxial à l'axe XX', constituant la partie (14,114) tibiale,
- Un montage (28,128) à roue libre comprenant un pignon (30,130) muni de rampes (32,132) et d'un rouleau (38,138) par rampe, ledit montage étant interposé entre la piste (20,120) du disque (16,116) et l'arbre (24,124) traversant unique,
- Des moyens (40,140) de déverrouillage comprenant une roue (42,142) à pions (44,144), montée également de façon coaxiale à l'axe XX', les pions (44,144) étant interposés entre la piste (20,120) et les rampes (32,132) du pignon (30,130), et
- Une partie (46,146) déportée des moyens (40,140) de déverrouillage comprenant des moyens (48, 148) de commande angulaire, **caractérisé en ce que** les moyens de commande angulaire sont constitués d'au moins une biellette (52,152) interposée entre ladite partie (46,146) déportée et la platine (14-1,114-1) tibiale.

2. Dispositif de verrouillage / déverrouillage d'une prothèse (10,110) de genou selon la revendication 1, **caractérisé en ce que** la biellette (52, 152) comprend une tête (54, 154) à une extrémité, la partie (46, 146) déportée comprend un logement (50, 150) rotulien borgne recevant ladite tête et **en ce que** la platine (14-1, 114-1) tibiale comprend un trou borgne (58, 158), destiné à recevoir de façon rigide la seconde extrémité de la biellette (52, 152).

3. Dispositif de verrouillage / déverrouillage d'une prothèse (10,110) de genou selon la revendication 1 ou 2, **caractérisé en ce que** le trou borgne (58,158) est orienté radialement par rapport à l'axe YY'.

4. Dispositif de verrouillage / déverrouillage d'une prothèse (10, 110) de genou selon l'une quelconque des revendications précédentes, **caractérisé en ce que** la platine (14-1, 114-1) tibiale est articulée par un bord distal autour de l'axe YY' autour d'un arbre (56, 156), disposé en avant de l'axe XX' par rapport au sens de la marche et traversant les deux joues (22-1, 22-2; 122-1, 122-2), gauche et droite.

5. Dispositif de verrouillage / déverrouillage d'une prothèse (10,110) de genou selon l'une quelconque des revendications précédentes, **caractérisé en ce qu'**il comprend une butée (60,160), réglable, solidaire des joues (22-1, 22-2; 122-1, 122-2), gauche et droite, disposée au droit du bord proximal de la platine (14-1, 114-1) tibiale de façon à limiter l'angle de pivotement de ladite platine (14-1, 114-1) vers le haut.

6. Dispositif de verrouillage / déverrouillage d'une prothèse (10, 110) de genou selon l'une quelconque des revendications précédentes, **caractérisé en ce qu'**il comprend des moyens (62, 162) amortisseurs et butée, de façon à éviter le mouvement angulaire du genou au-delà de l'alignement de la partie fémorale et de la partie tibiale.

7. Dispositif de verrouillage / déverrouillage d'une prothèse (10, 110) de genou selon l'une quelconque des revendications précédentes, **caractérisé en ce qu'**il comprend des billes de roulement disposées dans des cuvettes, en lieu et place de certains ensembles rampe (32, 132) rouleau (38, 138) du pignon (30, 130).

8. Dispositif de verrouillage / déverrouillage d'une prothèse (10, 110) de genou selon l'une quelconque des revendications précédentes, **caractérisé en ce que** l'arbre (24, 124) traversant comporte une cannelure (26, 126), **en ce que** le pignon (30, 130) à rampes (32, 132) du montage (28, 128) à roue libre comprend une cannelure (34, 134), et **en ce qu'**une clavette (36, 136) est interposée entre les deux cannelures de façon à assurer l'immobilisation angulaire dudit pignon (30, 130) à rampes (32, 132) sur ledit arbre (24).

9. Dispositif de verrouillage / déverrouillage d'une prothèse (10, 110) de genou selon l'une quelconque des revendications précédentes, **caractérisé en ce qu'**il comprend des moyens de rappel (53, 155) interposés entre la partie (12,112) fémorale et la partie (14, 114) tibiale, de façon à conserver un alignement entre les deux dites parties et à fournir une aide au retour.

10. Dispositif de verrouillage / déverrouillage d'une prothèse (10) de genou selon la revendication 9, **caractérisé en ce que** les moyens (155) de rappel comprennent un ressort à spires interposé sur l'entretoise (164), entre ladite entretoise et la pièce fémorale (112).

11. Dispositif de verrouillage / déverrouillage d'une prothèse (10) de genou selon la revendication 10, **caractérisé en ce que** la pièce fémorale (112) comprend une lumière curviligne (153) destinée à laisser passer la biellette (152).

12. Dispositif de verrouillage / déverrouillage d'une prothèse (10) de genou selon la revendication 11, **caractérisé en ce que** la lumière curviligne (153) comporte une butée réglable angulairement de façon à limiter l'amplitude.

13. Dispositif de verrouillage / déverrouillage d'une prothèse (10) de genou selon l'une quelconque des revendications précédentes, **caractérisé en ce qu'**il est prévu des moyens d'assistance au lever (66) à partir de la position assise comprenant un excentrique (68) apte à prendre deux positions :
- la première position (68-1) d'appui sur la biellette (62,162) des moyens de déverrouillage (40,140) pour assurer un déverrouillage permanent par les pions (44,144) qui repoussent chaque rouleau (38,138) en bas de sa rampe, et
- la seconde position (68-2), sans appui sur la tige (62,162) des moyens de déverrouillage (40,140), en laissant la biellette (62, 162) des moyens de verrouillage agir en fonctionnement normal.

## Patentansprüche

1. Verriegelungs-/Entriegelungsvorrichtung für eine Knieprothese (10, 110), einen Oberschenkelabschnitt (12, 112) umfassend, der zur Verbindung mit dem Oberschenkel des Prothesenträgers vorgesehen ist, und einen Schienbeinabschnitt (14, 114) mit einer sich um die Achse YY' drehenden Schienbeinplatine (14-1, 114-1), der ein künstliches Schienbein und einen künstlichen Fuß aufnehmen kann, wobei die beiden Abschnitte - Oberschenkelabschnitt (12, 112) und Schienbeinabschnitt (14, 114) - koaxial um eine Achse XX' drehend montiert sind und die Achsen XX' und YY' der natürlichen Beugungsachse des Knies entsprechend parallel zueinander sind, umfassend:
- ein den Oberschenkelabschnitt (12, 112) bildendes zylinderförmiges Teil (16, 116), mit einer zylinderförmigen, mittigen Aufnahme (18, 118), die koaxial zur Achse XX' ist und eine Lauffläche (20, 120) enthält,
- ein linkes und ein rechtes Seitenteil (22-1, 22-2; 122-1, 122-2) und eine einzige durchgehende, zur Achse XX' koaxiale Welle (24, 124), die den Schienbeinabschnitt (14, 114) bildet,
- eine Freilaufmontage (28, 128) mit einem Ritzel (30, 130), das mit Schlüsselnuten (32, 132) und einer Rolle (38, 138) pro Schlüsselnut versehen ist, wobei sich diese Montage zwischen der Lauffläche (20, 120) der Scheibe (16, 116) und der einzigen durchgehenden Welle (24, 124) befindet,
- Entriegelungsmittel (40, 140) mit einem ebenfalls koaxial zur Achse XX' gelagerten Stiftrad (42, 142) (44,144), wobei sich die Stifte (44, 144) zwischen der Lauffläche (20, 120) und den Schlüsselnuten (32, 132) des Ritzels (30, 130) befinden, und
- einen von den Entriegelungsmitteln (40, 140) entfernten Abschnitt (46, 146) mit Winkelsteuerungsmitteln (48, 148), **dadurch gekennzeichnet, dass** die Winkelsteuerungsmittel mindestens aus einer Schubstange (52, 152) bestehen, die zwischen dem entfernten Abschnitt (46, 146) und der Schienbeinplatine (14-1, 114-1) gelagert ist.

2. Verriegelungs-/Entriegelungsvorrichtung für eine Knieprothese (10, 110) nach Anspruch 1, **dadurch gekennzeichnet, dass** die Schubstange (52, 152) einen einendigen Kopf (54, 154) umfasst, und dass der entfernte Abschnitt (46, 146) eine blinde Kniescheibenaufnahme (50, 150) für diesen Kopf umfasst und dadurch, dass die Schienbeinplatine (14-1, 114-1) ein Sackloch (58, 158) für die starre Aufnahme des zweiten Endes der Schubstange (52, 152) umfasst.

3. Verriegelungs-/Entriegelungsvorrichtung für eine Knieprothese (10, 110) nach Anspruch 1 oder 2, **dadurch gekennzeichnet, dass** das Sackloch (58, 158) in Bezug auf die Achse YY' radial ausgerichtet ist.

4. Verriegelungs-/Entriegelungsvorrichtung für eine Knieprothese (10, 110) nach einem der vorausgehenden Ansprüche, **dadurch gekennzeichnet, dass** sich die Schienbeinplatine (14-1, 114-1) über einen distalen Rand um die Achse YY' um eine Welle (56, 156) dreht, die in Bezug zur Gehrichtung vor der Achse XX' angeordnet ist und das linke und rechte Seitenteil (22-1, 22-2; 122-1, 122-2) durchquert.

5. Verriegelungs-/Entriegelungsvorrichtung für eine Knieprothese (10, 110) nach einem der vorausgehenden Ansprüche, **dadurch gekennzeichnet, dass** sie einen mit dem linken und rechten Seitenteil (22-1, 22-2; 122-1, 122-2) fest verbundenen, einstellbaren Anschlag (60, 160) umfasst, der derart vor dem proximalen Rand der Schienbeinplatine (14-1, 114-1) angeordnet ist, dass der Schwenkwinkel der Platine (14-1, 114-1) nach oben hin begrenzt wird.

6. Verriegelungs-/Entriegelungsvorrichtung für eine Knieprothese (10, 110) nach einem der vorausgehenden Ansprüche, **dadurch gekennzeichnet, dass** sie Dämpfungs- und Anschlagmittel (62, 162) umfasst, sodass die Winkelbewegung des Knies über die geradlinige Ausrichtung des Oberschenkel- und Schienbeinabschnitts hinaus verhindert wird.

7. Verriegelungs-/Entriegelungsvorrichtung für eine Knieprothese (10, 110) nach einem der vorausgehenden Ansprüche, **dadurch gekennzeichnet, dass** sie anstelle von bestimmten Schlüsselnut-Rollen-Einheiten (32, 132) (38, 138) des Ritzels (30, 130) in Mulden angeordnete Lagerkugeln umfasst.

8. Verriegelungs-/Entriegelungsvorrichtung für eine Knieprothese (10, 110) nach einem der vorausgehenden Ansprüche, **dadurch gekennzeichnet, dass** die durchgehende Welle (24, 124) eine Keilnut (26, 126) umfasst, und dadurch, dass das Ritzel (30, 130) mit Schlüsselnuten (32, 132) der Freilaufmontage (28, 128) eine Keilnut (34, 134) umfasst, und dadurch, dass zwischen den zwei Keilnuten ein Spannkeil (36, 136) derart zwischengelagert ist, dass die winkelmäßige Blockierung des Ritzels (30, 130) mit Schlüsselnuten (32, 132) auf der Welle (24) gewährleistet ist.

9. Verriegelungs-/Entriegelungsvorrichtung für eine Knieprothese (10, 110) nach einem der vorausgehenden Ansprüche, **dadurch gekennzeichnet, dass** zwischen dem Oberschenkelabschnitt (12,112) und dem Schienbeinabschnitt (14,114) Rückzugmittel (53, 155) vorgesehen sind, sodass diese beiden Abschnitte fluchtend ausgerichtet bleiben und wieder leichter in ihre Ausgangspositionen zurückkehren können.

10. Verriegelungs-/Entriegelungsvorrichtung für eine Knieprothese (10, 110) nach Anspruch 9, **dadurch gekennzeichnet, dass** die Rückzugmittel (155) eine Spiralfeder umfassen, die zwischen dem Distanzstück (164) und dem Oberschenkelteil (112) gelagert ist.

11. Verriegelungs-/Entriegelungsvorrichtung für eine Knieprothese (10, 110) nach Anspruch 10, **dadurch gekennzeichnet, dass** der Oberschenkelteil (112) für den Durchgang der Schubstange (152) eine krummlinige Öffnung (153) umfasst.

12. Verriegelungs-/Entriegelungsvorrichtung für eine Knieprothese (10, 110) nach Anspruch 11, **dadurch gekennzeichnet, dass** die krummlinige Öffnung (153) einen hinsichtlich des Winkels einstellbaren Anschlag umfasst, um die Amplitude zu begrenzen.

13. Verriegelungs-/Entriegelungsvorrichtung für eine Knieprothese (10, 110) nach einem der vorausgehenden Ansprüche, **dadurch gekennzeichnet, dass** Hilfsmittel vorgesehen sind, um ausgehend von der sitzenden Stellung besser in die stehende Stellung (66) zu kommen, welche einen Exzenter (68) umfassen, der zwei Positionen einnehmen kann:
- die erste Position (68-1) mit Druck auf die Schubstange (62, 162) der Entriegelungsmittel (40, 140), um durch die Stifte (44, 144), die jede Rolle (38, 138) auf ihre Schlüsselnut hinabdrücken, eine permanente Entriegelung zu gewährleisten, und
- die zweite Position (68-2) ohne Druck auf die Schubstange (62, 162) der Entriegelungsmittel (40, 140), in der die Schubstange (62, 162) der Verriegelungsmittel in normaler Funktion agieren kann.

## Claims

1. A device for locking/unlocking a knee prosthesis (10, 110), comprising a femoral part (12, 112) designed to be connected to the thigh of the wearer, and a tibial part (14, 114) provided with a tibial plate (14-1, 114-1) articulated about an axis YY', able to receive an artificial tibia and foot, these two femoral (12, 112) and tibial (14, 114) parts both being mounted for rotation, coaxial about an axis XX', the axes XX' and YY' being parallel, corresponding to the bending axis of the natural knee, comprising:
- a cylindrical piece (16, 116) constituting the femoral part (12, 112), with a cylindrical central housing (18, 118), arranged coaxially with the axis XX' and comprising a track (20, 120),
- two cheeks (22-1, 22-2; 122-1, 122-2), left and right, and a single through shaft (24, 124), coaxial with the axis XX', constituting the tibial part (14, 114),
- a freewheel assembly (28, 128) comprising a pinion (30, 130) provided with ramps (32, 132) and a roller (38, 138) per ramp, said assembly being interposed between the track (20, 120) of the disc (16, 116) and the single through shaft (24, 124),
- unlocking means (40, 140) comprising a wheel (42, 142) with pins (44, 144), also mounted coaxially with the axis XX', the pins (44, 144) being interposed between the track (20, 120) and the ramps (32, 132) of the pinion (30, 130), and
- a part (46, 146) offset from the unlocking means (40, 140) comprising angular control means (48, 148), **characterised in that** the angular control means comprises at least one linkage (52, 152) interposed between said offset part (46, 146) and the tibial plate (14-1, 114-1).

2. A locking/unlocking device of a knee prosthesis (10, 110) according to claim 1, **characterised in that** the linkage (52, 152) comprises a head (54, 154) at one end, the offset part (46, 146) comprises a blind swivel housing (50, 150) receiving said head, and **in that** the tibial plate (14-1, 114-1) comprises a blind hole (58, 158) intended to rigidly receive the second end of the linkage (52, 152).

3. A locking/unlocking device of a knee prosthesis (10, 110) according to claim 1 or 2, **characterised in that** the blind hole (58, 158) is oriented radially with respect to the axis YY'.

4. A locking/unlocking device of a knee prosthesis (10, 110) according to any of the preceding claims, **characterised in that** the tibial plate (14-1, 114-1) is articulated by a distal edge about the axis YY' about a shaft (56, 156), disposed in front of the axis XX' with respect to the direction of travel and passing through the two cheeks (22-1, 22-2, 122-1, 122-2), left and right.

5. A locking/unlocking device of a knee prosthesis (10, 110) according to any of the preceding claims, **characterised in that** it comprises an adjustable stop (60, 160) secured to the left and right cheeks (22-1, 22-2; 122-1, 122-2), disposed in line with the proximal edge of the tibial plate (14-1, 114-1) so as to limit the angle of pivoting of said plate (14-1, 114-1) upwards.

6. A locking/unlocking device of a knee prosthesis (10, 110) according to any of the preceding claims, **characterised in that** it comprises damping and stop means (62, 162), so as to prevent the angular movement of the knee beyond the alignment of the femoral part and tibial part.

7. A locking/unlocking device of a knee prosthesis (10, 110) according to any of the preceding claims, **characterised in that** it comprises rolling balls disposed in cups, instead of some ramp (32, 132) and roller (38, 138) assemblies of the pinion (30, 130).

8. A locking/unlocking device of a knee prosthesis (10, 110) according to any of the preceding claims, **characterised in that** the through shaft (24, 124) comprises a groove (26, 126), **in that** the pinion (30, 130) with ramps (32, 132) of the freewheel assembly (28, 128) comprises a groove (34, 134), and **in that** a key (36, 136) is interposed between the two grooves so as to provide the angular immobilisation of said pinion (30, 130) with ramps (32, 132) on said shaft (24).

9. A locking/unlocking device of a knee prosthesis (10, 110) according to any of the preceding claims, **characterised in that** it comprises return means (53, 155) interposed between the femoral part (12, 112) and the tibial part (14, 114), so as to keep an alignment between said two parts and to provide assistance for return.

10. A locking/unlocking device of a knee prosthesis (10) according to claim 9, **characterised in that** the return means (155) comprise a coil spring interposed on the strut (164), between said strut and the femoral piece (112).

11. A locking/unlocking device of a knee prosthesis (10) according to claim 10, **characterised in that** the femoral piece (112) comprises a curved aperture (153) intended to allow the linkage (152) to pass.

12. A locking/unlocking device of a knee prosthesis (10) according to claim 11, **characterised in that** the curved aperture (153) comprises an angularly adjustable stop so as to limit the amplitude.

13. A locking/unlocking device of a knee prosthesis (10) according to any of the preceding claims, **characterised in that** means (66) are provided for assisting getting up from the seated position, comprising an eccentric (68) able to adopt two positions:
- the first position (68-1) of pressing on the linkage (62, 162) of the unlocking means (40, 140) for providing permanent unlocking by means of the pins (44, 144), which push each roller (38, 138) at the bottom of its ramp, and
- the second position (68-2), without pressing on the rod (62, 162) of the unlocking means (40, 140), allowing the linkage (62, 162) of the locking means to act in normal operation.
